# EUROPEAN PATENT APPLICATION

(11) **EP 1 884 221 A1**
(43) Date of publication of application: **06.02.2008**
(21) Application number: 07252477.0
(22) Date of filing: 19.06.2007
(51) Int. Cl.: A61F 2/38, A61B 17/72

(54) **Variable stiffness intramedullary system**

(30) Priority: 31.07.2006 US 461319
(71) Applicant: Zimmer Technology, Inc., Warsaw, IN 46580-2746 (US)
(72) Inventor: Crowninshield, Roy, Ft. Wayne IN 46814 (US); Wentz, Douglas, Warsaw IN 46580 (US); Stoller, Alex P., Warsaw IN 46580 (US)
(74) Representative: Findlay, Alice Rosemary

(57) **Abstract**

A variable stiffness stem component (10) for intramedullary fixation in total joint replacement implants or in a segmental replacement system. The stem component has a shaft (12) with a proximal end (16), a distal end (18), and a longitudinal length therebetween. The diameter of the shaft is approximately constant along the longitudinal length. A taper or threaded connection (14) may be provided adjacent the proximal end of the shaft for assembly to another implant component. At least three flutes (20) are disposed in a portion of the length of the shaft from intermediate the proximal and distal ends extending towards the distal end. The flutes increase in one of width or depth, or a combination thereof, towards the distal end to provide variable stiffness.

## Description

The present invention relates generally to devices for arthroplasty and bone segment replacement and specifically to intramedullary implants and stems.

Artificial joints are generally ball and socket or hinge joints designed to match as closely as possible the function of the natural joint. To duplicate a joint's natural motion, a total joint replacement implant often includes a bearing surface component, such as a spherical ball to replace the head of the femur, and a component which fits into the intramedullary canal of the femur, tibia, or humerus to provide stability for the bearing surface.

The stem component may also be used in the replacement of segments of the long bones of the upper or lower extremity. The bone segment application most likely occurs due to the treatment of bone tumors or cancer.

The presence of a stemmed prosthetic component in the intramedullary canal can be expected to change the distribution of stress from the joint to the adjacent skeleton. In a compound structure such as a prosthetic-bone structure, stress will tend to follow the stiffer pathway. The skeletal stiffening that results from the insertion of a relatively rigid prosthetic component can result in periprosthetic bone stress shielding. By way of explanation, where the weight load on the skeleton is large, the skeleton grows more bone tissue in the loaded area; the net result is a more closely packed and stronger skeleton that has the strength to sustain the increased load. In areas with diminished load, the skeleton retains only so much bone tissue as is necessary to sustain the diminished load. The skeleton in the diminished load areas is weaker. For example, in the case of total hip replacement, the stresses of the body weight flow through the total joint center, into the stem component and out of the stem component to the surrounding bone. A relatively stiff femoral stem component placed within a thighbone will share load with the surrounding bone. As a result of the load transfer through the prosthetic stem, the load in the surrounding bone is diminished. At the tip of the femoral stem component, load sharing and all stresses are transferred to the bone. There are two consequences of the changed flow of stresses associated with the prosthetic stem within a bone. First, due to reduced stress levels, the upper part of the thighbone may lose mass through a process known as osteopenia. As a result, the bone around the stem may become weaker and more susceptible to fracture. Second, the skeleton around the tip of the femoral stem component may experience locally high stresses as the load within the stem is transfer to the bone at the stem tip. Patients with total hip devices often complain about pain in the thigh or end of stem pain, especially during the first years after the surgery. Similar complaints of pain follow total knee replacement surgery, total shoulder replacement surgery, and bone segment replacement surgery. Based on the above explanation, it is believed that load sharing with the bone around a stemmed prosthetic component depends on the difference between the stiffness of the stem component and the stiffness of the bone.

Furthermore, periprosthetic pain can occur at the implant stem terminus as a result of the abrupt change in prosthetic stem reconstruction stiffness. In an attempt to alter the stiffness of a stem, a stem end "clothes pin" slot has been used to make a stem end more flexible. However, a split (one slot) stem will have an asymmetric bending stiffness and will affect stress development in the surrounding bone in a manner dependent upon the positioning of the split. Also, a single split will abruptly and substantially change bending stiffness and surrounding bone stress.

Thus, there is a need to control the structural stiffness of the terminus region of a prosthetic stem to provide a symmetrical transitional region of controllable load transfer to the surrounding bone. It is desired to reduce prosthesis-to-bone interface pressure and periprosthetic bone stress levels at a prosthetic stem terminus through implant design to provide reduced occurrence and severity of "end of stem pain" in a variety of prosthetic applications. It is further desired to control the stiffness through an implant design that also provides substantial surface area for stable fixation.

The present invention provides a variable stiffness stem component for intramedullary fixation in total joint replacement implants including knee, hip, and shoulder prosthesis and in a segmental replacement system. The stem component comprises a shaft having a proximal end, a distal end, and a longitudinal length therebetween. The shaft has an approximately constant diameter along the longitudinal length to provide maximal surface area for fixation. The stem component may further comprise a taper or threaded connection adjacent the proximal end of the shaft for assembly to another implant component. Alternatively, the proximal end of the shaft may integrally meet with another portion of a prosthetic element, for example in one-piece implants that do not include mechanical connection means between elements. The other end of the shaft, i.e. the distal end, extends away from the proximal end and is inserted within a bone. Variable stiffness is provided by a series of at least three flutes disposed in a portion of the length of the shaft from intermediate the proximal and distal ends and extending towards the distal end. The flutes increase in one of width or depth, or a combination thereof, towards the distal end. In one embodiment, the flutes may deepen to the extent that they produce a split distal stem with at least three split or discrete end portions.

The invention will now be further described by way of example with reference to the accompanying drawings in which:

FIG. 1 shows a side view of a stem according to certain embodiments of the invention.

FIGS. 2A-C show various cross sections of the stem of FIG. 1 that correspond by letter to cross sections indicated in FIG. 1.

FIG. 3 shows a side view of a stem according to certain embodiments of the invention.

FIGS. 4A-C show various cross sections of the stem of FIG. 3 that correspond by letter to cross sections indicated in FIG. 3.

FIG. 5 shows a stress analysis model for various stem designs.

FIG. 6 shows model analysis for tibial stress distribution for various stem designs.

The intramedullary implant stem component of the present invention provides intramedullary fixation when assembled with appropriate total joint replacement implants including knee, hip, and shoulder prosthesis. In other embodiments, the intramedullary implant stem component may be used in the replacement of segments of the long bones of the upper or lower extremity. The intramedullary implant stem component of the present invention has a variable stiffness approaching the stem terminus, which variable stiffness addresses potential problems such as stem tip pain and/or stress shielding associated with high stiffness stems or an abrupt change in stem stiffness.

In accordance with the present invention, the stem component comprises a shaft with a proximal end, a distal end, and a longitudinal length therebetween, wherein the shaft is of an approximately constant diameter along the longitudinal length. In one embodiment, the shaft terminates at the proximal end with a taper or threaded portion. The taper or threaded portion is a connection means that allows for modular assembly to total joint implants designed for the proximal tibia, distal femur, proximal femur, or proximal humerus. In another embodiment, the shaft terminates at the proximal end by integrally meeting another element of the implant without mechanical connection means. To provide the variable stiffness, the stem has a unique geometry of flutes that are distal to the proximal end. The flutes extend along a portion of the shaft and the flute dimensions widen and/or deepen in a continuous fashion as they progress towards the distal end. The flutes may deepen to the extent that they produce a split distal stem with at least three split or discrete end portions. This geometry produces a continuous decrease in bending stiffness of the stem with no abrupt or discontinuous changes in bending stiffness. Further, the flute geometry maintains a substantial percentage of stem material at the nominal stem geometry, such that primary fixation of the stem (e.g. torsional stability) is not compromised.

The terms "distal" and "proximal" by definition refer to a location further from or nearer to, respectively, a reference point. The reference point may vary in different fields, e.g. medical and mechanical. For example, in the medical field, the reference point may be the body midline, or mesial plane. The reference point could also refer to a point of attachment whether the attachment is mechanical or non-mechanical. Herein, to avoid a change of meaning of these terms based on the location or orientation of the implant in the body, proximal shall refer to being next to or nearest the point of attachment, or the point at which the shaft integrally meets another element of the implant device. Specifically, the reference point is the taper or threaded connection in a modular assembly-type implant, or in a one-piece implant, the integral meeting point of the shaft with the remainder of the implant. Similarly, distal shall refer to being situated away from or furthest from the reference point.

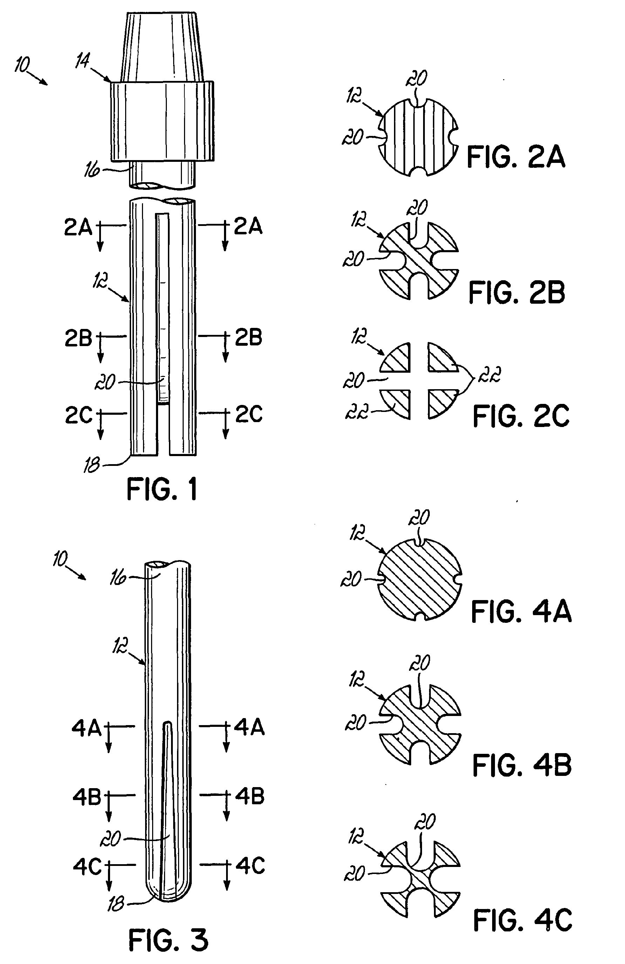

The component will now be explained with reference to the figures wherein like reference numerals are used to refer to like parts throughout the several views. As shown in FIG. 1, an intramedullary implant stem component 10 has a shaft 12 with a longitudinal length and an upper stem portion 14. In the embodiment shown, the upper stem portion 14 has a tapered or threaded portion to provide a connection means that allows for modular assembly to total joint implants designed for the proximal tibia, distal femur, proximal femur, or proximal humerus, or to a segmental replacement implant.

The shaft 12 has a proximal end 16 where the shaft 12 adjoins the upper stem portion 14 and an opposing distal end 18. The distal end 18 is adapted to be inserted into a patient's intramedullary canal to secure the stem in place and it may be flat, rounded, bullet-nosed, or any other useful configuration. In an exemplary embodiment, the cross section of the shaft 12 may be substantially circular. The shaft 12 may be substantially straight or curved, for example, such that it matches the curvature of the anatomy, for example the femur. A distal portion of the length of shaft 12 is shown having a series of flutes 20. In accordance with the present invention, there are at least three flutes 20. In one embodiment, the flutes 20 are substantially equidistant from one another and are substantially parallel to the longitudinal axis of the shaft 12. The series of flutes 20 is provided, among other things, for variable bending stiffness. Furthermore, the multiple flutes 20 provide a substantially axisymmetrical reduction in bending stiffness of the distal stem. An axisymmetrical bending stiffness does not require a surgeon to orient the stem based on possible directions of force application or bending. Therefore, flute geometries that create an axisymmetrical bending stiffness are disclosed.

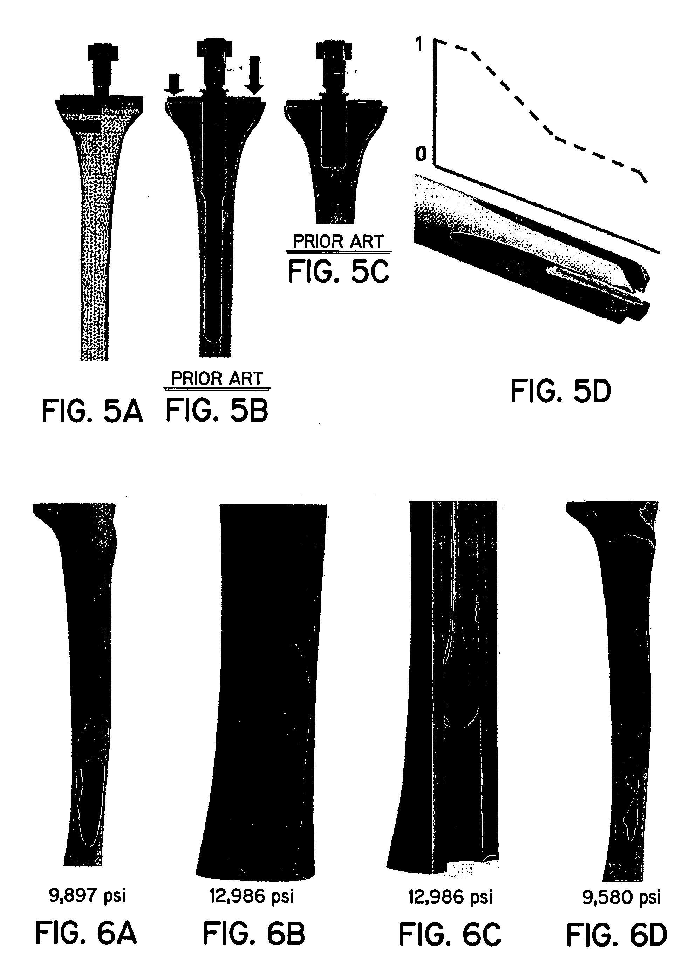

FIGS. 2A-C each show a series of four flutes 20 on the stem component 10 that are formed in a circular cross-sectional shape. However, the cross sectional shape may also be triangular, square, or other. In one embodiment, as shown by progressively viewing FIGS. 2A, 2B, and 2C, as the flutes 20 progress towards the distal end 18, they become deeper while maintaining a substantially constant width. In another embodiment, shown in FIG. 3 and by progressively viewing FIGS. 4A, 4B, and 4C, the flutes 20 become both deeper and wider as they progress towards the distal end 18. In another embodiment, the flutes 20 become wider while maintaining a relatively constant depth as they progress towards the distal end. In one embodiment, the flutes 20 may join at a point proximal to the distal end 18, as shown in FIG. 1, or approximately at the distal end 18. Alternatively, the flutes 20 may not join, as shown in FIG. 4C. When the stem is configured such that the flutes 20 intersect, the result is a split end having a plurality of distal end portions 22, as shown in FIG. 2C, which are discrete from one another at the distal end 18. These discrete portions 22 may also be referred to as creating terminus slots, the number of slots being equal to the number of flutes 20. The terminus slots may have the same width as the flutes, as shown in FIG. 2C. Alternatively, the terminus slots may have a different width than the flutes, as shown in FIG. 5D.

The flutes 20 are disposed in a portion of the length of the shaft 12 beginning from a location intermediate the proximal end 16 and the distal end 18 and extending toward the distal end 18. In certain embodiments, flutes 20 having the described configurations may extend the substantial length of the shaft 12 or any part of the shaft 12. In one embodiment, the flutes 20 begin at approximately the middle of the shaft 12.

The unfluted portion of the shaft 12 defines an outer profile of the stem shaft, the diameter of which is referred to as the nominal shaft diameter. The diameter in the fluted portion of shaft 12 is substantially equal to the nominal shaft diameter, such that the diameter of shaft 12 is substantially constant along the length of the shaft 12, in both the fluted and unfluted portions. By substantially maintaining the diameter throughout the length of the shaft 12, maximal contact between the stem 10 and the bone is maintained, thus resulting in maximal stability of the implant. In one embodiment, the outer profile is circular or substantially circular and approximates the cross section of the site of implantation following preparation of the bone, for example following reaming of the bone's intramedullary cavity. However, in other embodiments the outer profile may include other geometries, with the outer profile dimension being maintained substantially constant throughout the length of the shaft.

The stem component 10 may be made from any biocompatible material that has sufficient strength to withstand the patient's weight, examples of which include titanium, titanium alloys, stainless steel, stainless steel alloys, cobalt alloys, and other surgical grade material.

The surface of stem component 10, and in particular the surface of shaft 12, may be provided with a bone in-growth or on-growth feature. This may be a surface of raised splines, a roughened surface, metallic beads, a grit blasted surface, a porous surface, a hydroxyapatite coating, or any other bone growth-promoting substance coating, or combinations of any of these features. This allows the bone into which the stem is implanted to integrate with the stem 10 or otherwise grow into the flutes 20 for increased strength and stability.

In use, the variable stiffness stem helps provide stability to a bone or joint replacing prosthesis component. A surgical method for using the variable stiffness stem of the present invention includes preparing the implantation site for the prosthesis by surgically exposing the site for prosthesis implantation, followed by resection of the bone or joint segment to be replaced. The adjacent portions of bone are then prepared to receive an intramedullary prosthesis stem. This bone preparation can include the reaming of the bone's intramedullary cavity, measuring or sizing the cavity, and/or the removal of bone cavity contents. The bone preparation may be assisted by the use of radiographic imaging or alignment instruments. The variable stiffness stem of desired cross-sectional size and length can then be inserted into the prepared bone cavity. Prior to insertion into the bone, the stem may be attached to other prosthesis elements or it can be attached after insertion. Alternatively, the stem may be an integral part of a one-piece implant device.

### EXAMPLE

The following example is provided to illustrate the invention and is not intended to limit the same.

Three dimensional, finite element stress analysis models of the proximal tibia with total knee replacement (TKR) implants subjected to joint loading associated with a constrained implant in mid-stance gait (FIGS. 5A-D) were developed. As depicted in FIG. 5A, the models were constructed utilizing approximately 130,000 tetrahedral and 35,000 contact elements. The bone of the proximal tibia was modeled as a linearly elastic material with a spatial distribution of the modulus of elasticity of trabecular bone. The prosthesis-to-bone interface was modeled as bonded under the tibial tray and as press fit along the stem with an experimentally determined coefficient of friction. The implant components were modeled as constructed of cobalt alloy. Several tibial stem component design features were parametrically modeled ranging from a 175 mm solid cylindrical stem (FIG. 5B), a 35 mm primary stem component (FIG. 5C), and a 175 mm variable stiffness stem with flutes transitioning into multiple stem terminus slots (FIG. 5D).

The first principal stress within the cortical bone at the stem tip and the maximum pressure between the prosthetic stem tip and surrounding bone were predicted to vary greatly with implant design. As shown in FIGS. 6A and 6B, and as set forth numerically in Table 1, the use of a solid 175 mm long stem compared to a 35 mm stem tibial tray increased the maximum stress within the tibial cortex by 34% while tripling the pressure between the bone and implant at the stem tip. The incorporation of a transitional, reduced stiffness region in a 175 mm stem, in accordance with the present invention, reduced the maximum bone stress levels to near that of the 35 mm stem implant while reducing the pressure between the stem tip and bone by 49%.

**Table 1**

| | Maximum Bone Stress (ksi) | Maximum Stem Interface Pressure (ksi) |
|---|---|---|
| 35 mm stem | 9.9 | 1.5 |
| Solid 175mm stem | 13 | 4.7 |
| Transitional Stiffness 175 mm stem | 9.6 | 2.4 |

The distribution of stress within the tibia was greatly effected by prosthetic component design. FIG. 6 shows the tibial cortex first principal stress distribution and maximum values. FIG. 6A shows the first principal stress on the lateral tibial cortex surface associated with a 35 mm stem tray with the maximum pressure indicated below the model. FIGS. 6B and 6C (cut away view) shows the tibial stress concentration that occurs at the stem tip as load is transferred from the solid 175 mm stem and the resulting maximum pressure. FIG. 6D shows the reduction in tibial stress concentration associated with the 175 mm variable stiffness stem of the present invention.

The use of rigid intramedullary stem prosthesis in many joint reconstruction situations can be associated with end of stem pain that limits patient function. This pain may be the result of the localized load transfer, high bone stress development, and high implant-to-bone pressure that occurs at the stem tip. The above analysis demonstrates that implant design features that alter stem terminus bending stiffness can significantly alter the periprosthetic bone loading condition to an extent that stress induced periprothetic pain and function limitation may be reduced. The multiple flute stem geometry of the present invention has a nearly axisymmetric bending stiffness and can provide for smooth, continuous, and substantial bending stiffness reduction that results in less localized load transfer and stress concentration at the stem tip.

Other variations or embodiments of the invention will also be apparent to one of ordinary skill in the art from the above description and example.

## Claims

1. An intramedullary implant stem component, comprising:
(a) a shaft having a proximal end, a distal end, and a longitudinal length therebetween, wherein the shaft has an approximately constant diameter along the longitudinal length; and
(b) a series of at least three flutes disposed in a portion of the length of the shaft from intermediate the proximal and distal ends extending towards the distal end, wherein the flutes increase in one of width or depth, or a combination thereof, towards the distal end.

2. The intramedullary implant stem component of claim 1 wherein the flutes join at a location proximal the distal end to form discrete end portions at the distal end.

3. The intramedullary implant stem component of either claim 1 or claim 2 wherein the flutes extend from approximately the middle of the shaft to the distal end.

4. The intramedullary implant stem component of any preceding claim wherein the shaft includes a bone on-growth or bone in-growth feature comprising a raised splined surface, a roughened surface, metallic beads, a grit blasted surface, a porous surface, or a hydroxyapatite coating, or combinations thereof.

5. The intramedullary implant stem component of any preceding claim, wherein the stem is curved.

6. The intramedullary implant stem component of any preceding claim wherein the flutes are arranged equidistant from one another on the shaft.

7. The intramedullary implant stem component of any preceding claim wherein the flutes continuously increase in depth as the flutes extend toward the distal end.

8. The intramedullary implant stem component of any preceding claim wherein the flutes continuously increase in depth and width as the flutes extend toward the distal end.

9. The intramedullary implant stem component of any preceding claim wherein a tapered or threaded connection is adjacent the proximal end of the shaft.

10. The intramedullary implant stem component of any preceding claim wherein the proximal end integrally meets with a remaining portion in a one-piece joint implant.

11. The intramedullary implant stem component of any one of claims 1 to 8 further comprising connection means adjacent the proximal end of the shaft for attachment to a joint implant.
